# EUROPÄISCHE PATENTSCHRIFT

(11) **EP 0 401 657 B1**
(45) Veröffentlichungstag und Bekanntmachung des Hinweises auf die Patenterteilung: **19.01.1994**
(21) Anmeldenummer: 90110240.0
(22) Anmeldetag: 30.05.1990
(51) Int. Cl.: C12P 21/02, C07K 3/08

(54) **Verfahren zur C-terminalen Modifizierung von Proteinen**
Process for modifying the C-terminal end of proteins
Procédé pour modifier l'extrémité C-terminale des protéines

(30) Priorität: 03.06.1989 DE 3918125
(43) Veröffentlichungstag der Anmeldung: 12.12.1990
(73) Patentinhaber: FORSCHUNGSZENTRUM JÜLICH GMBH, 52425 Jülich (DE)
(72) Erfinder: Schwarz, Alexander, D-5000 Köln 60 (DE); Wandrey, Christian, Prof. Dr., D-5170 Jülich (DE); Wilchek, Meir, Rehovot (IL); Bayer, Edward Allen, Raanana (IL)

(56) Entgegenhaltungen:
- CHEMICAL ABSTRACTS, Band 111, Nr. 5, 31. Juli 1989, Columbus, OH (US); L. FRITZ et al., Seite 76, Nr. 33725d#
- CHEMICAL ABSTRACTS, Band 108, Nr. 9, 29. Februar 1988, Columbus, OH (US); D.J. GWYNFOR et al., Seite 539, Nr. 73676h#
- CHEMICAL ABSTRACTS, Band 106, Nr. 21, 25. Mai 1987, Columbus, OH (US); C. CARLES et al., Seite 371, Nr. 172365w#
- CHEMICAL ABSTRACTS, Band 97, Nr. 13, 27. September 1982, Columbus, OH (US); K. BREDDAM et al., Seiten 650-651, Nr. 110372h#

## Beschreibung

Die Erfindung bezieht sich auf ein Verfahren zur C-terminalen Modifizierung von Protein mit zumindest einem chemisch reaktiven oder erkennungsspezifischen Modifikator, der mit dem Protein über eine L-Aminosäure oder ein L-Aminosäurederivat als Kopplungsglied durch Amidbildung verknüpft ist.

Verfahren zur Modifizierung von Proteinen mit bestimmten Funktionen wie Markierungs-, Immobilisierungs- oder Vernetzungsgruppen sind bekannt, bei denen eine mehr oder minder zufällige Modifikation über Lysingruppen im Protein erreicht wird. Auch andere Gruppen in Proteinen wie Tyrosin, Cystein werden durch diese Verfahrensweise ungezielt modifiziert.

Dabei werden schwerlich eindeutig modifizierte Produkte erhalten, und die Aktivität des modifizierten Proteins ist im allgemeinen erheblich vermindert.

Ein Prinzip zur C-terminalen Modifizierung von Proteinen wird von K. Rose et al. (Europ. Peptid-Symp. Tübingen, BRD, Sept. 1988, Poster Nr. 64) angegeben, die am C-Terminus mit Hilfe proteolytischer Enzyme ein Kopplungsglied anfügen, an das dann in einem zweiten Schritt ein Modifikator gebunden wird.

Über eine C-terminale Modifizierung von Protein wird auch bereits von K. Hofmann et al. (J. Am. Chem. Soc. 1978, S. 3585 f.) berichtet, die allerdings durch chemische Umsetzungen herbeigeführt wird.

Diese Verfahrensweisen zur C-terminalen Modifizierung von Protein sind relativ aufwendig.

Ziel der Erfindung ist daher ein Verfahren, mit dem eine C-terminale Modifikation eines Proteins relativ einfach erreicht werden kann.

Das zu diesem Zweck entwickelte erfindungsgemäße Verfahren der eingangs genannten Art ist dadurch gekennzeichnet, daß man das Protein mit der zumindest einen Modifikator tragenden Aminosäure bzw. dem Aminosäurederivat mit freier α-Aminogruppe enzymatisch in Gegenwart von Protease zum C-terminal ein- oder mehrfach modifizierten Protein umsetzt.

Zwar ist die enzymatische C-terminale Anknüpfung von Aminosäuren oder auch von Aminosäureamiden oder -estern an Peptide in Gegenwart von Protease, insbesondere Carboxypeptidase Y, seit einiger Zeit bekannt (US-PS 4,339,534), jedoch wurde diese enzymatische Reaktion bislang nicht zur C-terminalen Anknüpfung von chemisch reaktiven Modifikatoren oder komplizierten erkennungsspezifischen Verbindungsgruppen benutzt.

Proteolytische Enzyme wie Trypsin oder CPD-Y wurden bislang lediglich für C-terminale Transpeptidierungen (K. Morihara et al., Biochem. J. 240 (1986) 803; Nature 280 (1979) 412 sowie K. Breddam et al., Carlsberg Res. Commun. 46 (1981) 121) sowie für Polypeptidsynthesen (z.B. K. Rose et al., Biochem. J. 249 (1988) 83 - 88) verwendet.

Eine Ankopplung von chemisch reaktiven oder relativ komplizierten Modifikatoren an Proteine, wie sie gemäß R. E. Feeny, Int. J. Peptide Protein Res. 29, 1987, 145 - 161, seit 40 Jahren in stürmischer Entwicklung befindlich ist, durch einfache enzymatische Umsetzung zu erreichen, ist jedoch bislang nicht unternommen worden.

Gemäß der Erfindung wird nun (zumindest) ein Modifikator gezielt am C-Terminal des Proteins angekoppelt, das so auf eindeutige Weise durch eine Markierungs-, Immobilisierungs-, Aktivierungs-u.dgl.-Funktion abgewandelt wird. Als Kopplungsglied dient dabei insbesondere ein zumindest trifunktionelles Aminosäurederivat,dessen Dritt-(oder Mehr-)funktion durch eine Amino- Thiol-, Hydroxy-, Carboxy- o.dgl.-Gruppe gebildet werden kann. Beispiele für Aminosäuren mit Drittfunktion, die als Kopplungsglieder benutzt werden können, sind insbesondere Lysin, Cystein Glutaminsäure, Asparaginsäure usw.

Für die erfindungsgemäße enzymatische C-terminale Proteinmodifikation kann aber auch eine einfache Aminosäure verwendet werden, über deren Carboxy-Funktion der Modifikator angekoppelt ist. Analog kann für die Modifikation eine trifunktionelle Aminosäure dienen, die einen oder zwei (gleiche oder unterschiedliche) Modifikator(en) aufweist, die über die Drittfunktion und/oder die Carboxy-Funktion angekoppelt sind. Falls die Carboxyfunktion nicht besetzt ist, wird für die enzymatische Umsetzung ein Säurederivat, insbesondere ein Säureamid verwendet.

Spezielle Kopplungsglieder mit Modifikatoren X für eine singuläre Modifikation entsprechen insbesondere den Formeln:

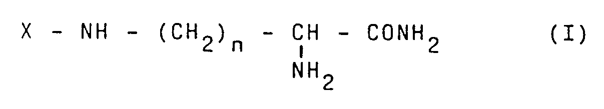

mit n = 1 - 10

oder

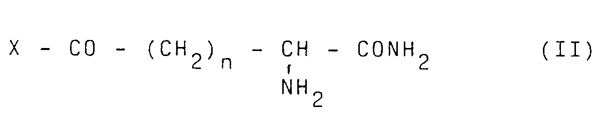

mit n = 1 - 10

oder

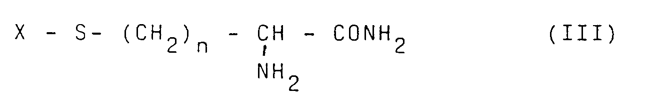

mit n = 1 - 10.

Bei Verbindungen der vorstehenden Formeln I bis III sowie bei Verbindungen der Formel V können die Modifikatoren X noch durch einen weiteren, vor der Koppelfunktion eingeschobenen Spacer von der an das Protein enzymatisch ankoppelnden Aminofunktion entfernt sein, z. B. unter Bildung von

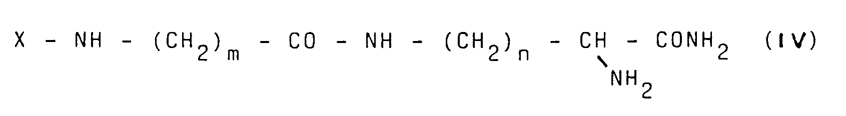

mit m = 1 bis 10

Bei diesen (ggf. verlängerten) Kopplungsgliedern der vorstehenden Formeln I bis III können aber auch die Amidreste durch einen weiteren Modifikator ersetzt sein oder einen solchen aufweisen. Die mit dem Protein umzusetzenden Verbindungen entsprechen dann z. B. der Formel: 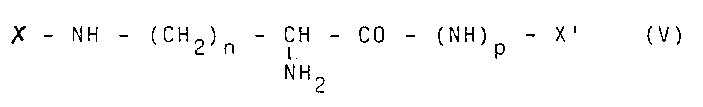
wobei die Modifikatoren X und X' gleich oder verschieden sein können, p = 1 oder 0 ist und n die bereits angegebene Bedeutung hat.

Ein über die Carboxyfunktion angekoppelter Modifikator kann aber auch über eine einfache L-Aminosäure C-terminal an das Protein angeheftet werden, unter Verwendung von Aminosäuren der allgemeinen Formel: 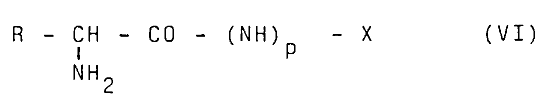
Als Modifikatoren X bzw. X' können z. B. Biotin, Maleimidoalkansäure, Pyridyldisulfid, Phenylazid usw. unter Amid-, Ester- oder Thioester-Bildung oder dergleichen angekoppelt werden.

Nach ihren unterschiedlichen Anwendungsbereichen kann man folgende Modifikatoren unterscheiden:
- als Markierungsgruppen, z. B. Biotin
- als Fluororescenzmarker, z. B. Dansyl-, Dabsyl-, Fluororescin
- als Vernetzungsreagentien, z. B. Maleimido-, 2-Pyridyl-disulfid, (4-Jodoacetyl)-aminobenzoat
- als photoreaktive Vernetzer, z. B. 4-Azidosalicylsäure, 2-(p-Azidosalicylamido)-ethyl-1,3'-dithiopropionat.

Die Umsetzung erfolgt in wäßrig organischer Lösung, wobei als organische Komponente polare Lösungsmittel wie insbesondere Dimethylsulfoxid oder Dimethylformamid infrage kommen Protein zu Proteaseverhältnisse von 10 : 1 bis 100 : 1 sind besonders geeignet und das anzukoppelnde Nukleophil wird in großem Überschuß verwendet. Der pH-Wert sollte im Bereich von 7 bis 10, insbesondere bei etwa 8,5 liegen. Die Umwandlungstemperatur sollte möglichst niedrig sein und Zimmertemperatur nicht überschreiten, insbesondere wird unter Kühlung der Lösung gearbeitet, vorzugsweise bei etwa 4°C. Die Umwandlungsdauer beträgt einige Std. Die erhaltene Reaktionsmischung wird durch Chromatographie, Gelfiltration und ggf. Aufkonzentration des erhaltenen modifizierten Proteins aufgearbeitet.

Zum Nachweis der Tauglichkeit des erfindungsgemäßen Verfahrens wurden insbesondere durch Biotin bzw. Maleimidopropionyl C-terminal modifizierte Proteine als Beispiel hergestellt:

### Beispiel 1:

### Herstellung von C-terminal biotinylierten Proteinen.

ε-Biotinyl-L-lysin-amid (Biocytinamid) - hergestellt nach einer modifizierten Vorschrift von Hoffmann et al, J. Am. Chem. Soc. 100 (1978) 3585 - wurde in einer Konzentration von 100 mg/ml in einer Mischung von Wasser, DMF und Ethanol (2 : 1 : 1) gelöst. Der pH-Wert wurde mittels 5 M NaOH auf 8,5 eingestellt. Zu dieser Lösung wurden Zielprotein und CPD-Y in einem molaren Verhältnis von 100/1 bzw. 50/1 gegeben und die Reaktion über Nacht bei 4°C ablaufen gelassen.

Die Reaktionsmischung wurde dann über eine Sephadex® G-25 Eine Säule mit 1 M Essigsäure als Eluent aufgetrennt und die Proteinfraktion gesammelt.

Die Lösung wurde ggf. durch Lyophilisierung oder Ultrafiltration aufkonzentriert.

### Charakterisierung:

Die Inkorporation von Biotin in das Protein konnte durch dotblots angefärbt mit Avidin komplexierter Alkalinen Phosphatase gezeigt werden (M. Wilchek et al, Biochem. Biophys. Res. Commun., 138 (1986) 872). Dies C-terminal biotinylierte Protein ist durch eine einzelne Bande im nativen PAGE und Blot transfer auf Nitrocellulose-Membran charakterisiert; es wandert an die gleiche Stelle wie das entsprechende chemisch biotinylierte Protein. Dies weist darauf hin, daß die Proteine unter diesen Reaktionsbedingungen nicht weiter hydrolisiert werden.

Folgende Proteine konnten erfolgreich biotinyliert werden: Insulin, Trypsin, Myoglobin, Cytochrome-C, RNAase, Lysozym, BSA und menschliches Albumin.

### Beispiel 2:

### C-terminale Maleimidopropionylierung von Proteinen.

1 g Maleimidopropionylhydroxysuccinimidester wurde in trockenem DMF gelöst und die Lösung mit 2,4 g α-BOC-L-Lys-NH₂ versetzt. Die Reaktionsmischung wurde 24 h bei Raumtemperatur gerührt. Durch Zugabe von trockenem Ether wurde α-BOC-ε-maleimidopropionyl-L-Lys-NH₂ ausgefällt. Der Niederschlag wurde abfiltriert, mit trockenem Ether gewaschen und die α-BOC-Schutzgruppe dann mittels 4 N HCl in Dioxan abgetrennt.

Das erhaltene Maleimidopropionyllysinamid wurde in analoger Weise wie das Biocytinamid gemäß Beispiel 1 enzymatisch mit Proteinen zu den entsprechenden C-terminal modifizierten Proteinen umgesetzt.

### Charakterisierung.

Die modifizierten Proteine (Insulin, Myoglobin Lysozyme und Cytochrome-C) und die entsprechenden nichtmodifizierten Proteine wurden mit Mercaptoethanol behandelt. Der Überschuß an Mercaptoethanol wurde mit Ellmann's Reagenz zurücktitriert. Zum Vergleich wurden nichtmodifizierte Proteine ebenso mit Mercaptoethanol behandelt und die Rücktitration in gleicher Weise vorgenommen. Alle modifizierten Proteine zeigten eine geringere Färbung als die nichtmodifizierten.

Der Grad der Modifizierung wurde mittels Immobilisierung an SH-modifiziertem Eupergit® abgeschätzt. Hierzu wurden 40 mg SH-modifizierter Eupergit® mit 1 ml des modifizierten Proteins (c = 0,25 mg/ml) bei Zimmertemperatur und pH 7,8 eine Stunde geschüttelt. Nach Zentrifugation wurde der Überstand gesammelt und die Kugeln mit Mercaptoethanol behandelt. Nach neuerlicher Zentrifugation wurde die optische Dichte des Überstandes vor der Immobilisierung und nach der Immobilisierung gemessen. Hierbei lagen die geschätzten Grade der Modifizierung zwischen 75 % bei Cytochrome-C und 40 % bei Insulin.

Gemäß der Erfindung C-terminal modifizierte Proteine können ohne Schwierigkeit miteinander gekoppelt werden:
Die Kopplung zweier Proteine über ihre C-Termini gelang durch Zugabe von DTT zu einer Lösung von modifiziertem Myoglobin bei pH 7,5 und einstündiges Schütteln. Der Nachweis der Verdoppelung des Molekulargewichtes von ca. 17.000 auf ca. 34.000 erfolgte durch native PAGE mit entsprechenden Molekularmarkern.

## Patentansprüche

1. Verfahren zur C-terminalen Modifizierung von Protein mit zumindest einem chemisch reaktiven oder erkennungsspezifischen Modifikator, der mit dem Protein über eine L-Aminosäure oder ein L-Aminosäurederivat als Kopplungsglied durch Amidbildung verknüpft ist,
**dadurch gekennzeichnet,**
daß man das Protein mit der zumindest einen Modifikator tragenden Aminosäure bzw. dem Aminosäurederivat mit freier α-Aminogruppe enzymatisch in Gegenwart von Protease zum C-terminal ein- oder mehrfach modifizierten Protein umsetzt.

2. Verfahren nach Anspruch 1,
**dadurch gekennzeichnet,**
daß man als Protease Carboxypeptidase Y verwendet.

3. Verfahren nach Anspruch 1 oder 2,
**dadurch gekennzeichnet,**
daß man ein trifunktionelles L-Aminosäurederivat mit über die Drittfunktion und/oder Carboxyfunktion angekoppeltem Modifikator (bzw. Modifikatoren) verwendet.

4. Verfahren nach Anspruch 3,
**dadurch gekennzeichnet,**
daß man ein trifunktionelles L-Aminosäurederivat mit zwei unterschiedlichen über die Dritt- und die Carboxyfunktion angekoppelten Modifikatoren verwendet.

5. Verfahren nach Anspruch 3,
**dadurch gekennzeichnet,**
daß man ein trifunktionelles L-Aminosäureamid mit über die Drittfunktion angekoppelten Modifikator verwendet.

6. Verfahren nach Anspruch 5,
**dadurch gekennzeichnet,**
daß man das Protein mit einem ε-N-modifizierten L-Lysinamid umsetzt.

## Claims

1. A process for the terminal C modification of a protein with at least one chemically reactive or identification-specific modifier which is linked to the protein by amide formation via an L-amino acid or an L-amino acid derivative as the coupling element, characterized in that the protein is enzymatically reacted with the amino acid or the amino acid derivative with a free α-amino group, and which comprises at least one modifier, in the presence of a protease, to form a protein with a singly- or multiply-modified terminal C.

2. A process according to claim 1, characterized in that carboxypeptidase Y is used as the protease.

3. A process according to claim 1 or 2, characterized in that a trifunctional L-amino acid derivative is used, with a modifier (or modifiers) coupled via the third function and/or carboxy function.

4. A process according to claim 3, characterized in that a trifunctional L-amino acid derivative is used, with two different modifiers coupled via the third and the carboxy functions.

5. A process according to claim 3, characterised in that a trifunctional L-amino acid amide is used, with a modifier coupled via the third function.

6. A process according to claim 5, characterized in that the protein is reacted with an ε-N-modified L-lysinamide.

## Revendications

1. Procédé pour la modification C-terminale d'une protéine avec au moins un modificateur chimiquement réactif ou spécifique d'identification, qui est combiné avec la protéine par l'intermédiaire d'un amino-acide L ou d'un dérivé d'amino-acide L, grâce à la formation d'un amide, caractérisé en ce qu'on fait réagir la protéine avec l'amino-acide ou avec le dérivé d'amino-acide portant au moins un modificateur et ayant un groupe amino α libre, par voie enzymatique en présence d'une protéase, pour la transformer en une protéine avec une modification C-terminale simple ou multiple.

2. Procédé selon la revendication 1, caractérisé en ce qu'on utilise, comme protéase, une carboxypeptidase Y.

3. Procédé selon la revendication 1 ou 2, caractérisé en ce qu'on utilise un dérivé trifonctionnel d'amino-acide L avec un modificateur (ou des modificateurs) couplé par l'intermédiaire de la fonction troisième et/ou de la fonction carboxy.

4. Procédé selon la revendication 3, caractérisé en ce qu'on utilise un dérivé trifonctionnel d'amino-acide L avec deux modificateurs différents couplés par l'intermédiaire de la fonction troisième et de la fonction carboxy.

5. Procédé selon la revendication 3, caractérisé en ce qu'on utilise un amide trifonctionnel d'amino-acide L avec un modificateur couplé par l'intermédiaire de la fonction troisième.

6. Procédé selon la revendication 5, caractérisé en ce qu'on fait réagir la protéine avec un amide de L-lysine modifié en ε-N.
